# EUROPÄISCHE PATENTANMELDUNG

(11) **EP 1 477 225 A1**
(43) Veröffentlichungstag der Anmeldung: **17.11.2004**
(21) Anmeldenummer: 04010544.7
(22) Anmeldetag: 04.05.2004
(51) Int. Cl.: B01J 29/89, B01J 21/08, C07D 301/12

(54) **Katalysator**

(30) Priorität: 15.05.2003 DE 10321876
(71) Anmelder: Bayer MaterialScience AG, 51368 Leverkusen (DE)
(72) Erfinder: Weisbeck, Markus, Dr., 51067 Köln (DE); Heinen, Marie-Therese, 42929 Wermelskirchen (DE); Schmitt, Jörg, 41515 Grevenbroich (DE); Wegener, Gerhard, Dr., 40822 Mettmann (DE); Dugal, Markus, Dr., 47906 Kempen (DE)

(57) **Zusammenfassung**

Die vorliegende Erfindung betrifft einen Katalysator enthaltend Titan in gebundener Form, nichtkristallines Siliziumdioxid und mindestens eine kristalline Silikatphase, die eine Zeolithstruktur aufweist, wobei das nichtkristalline Siliziumdioxid auf mindestens einer der kristallinen Silikatphasen, die eine Zeolithstruktur aufweisen, aufgebracht ist, und wobei mindestens eine der kristallinen Silikatphasen, die eine Zeolithstruktur aufweisen, Silizium-Kohlenstoff-Bindungen enthält, mit denen nicht hydrolytisch abtrennbare organische Gruppen R an Silizium gebunden sind. Weiterhin betrifft die vorliegende Erfmdung ein Verfahren zur Herstellung dieses Katalysators und ein Verfahren zur Herstellung eines Epoxides aus einer Verbindung, die eine Kohlenstoff-Kohlenstoff-Doppelbindung enthält (bevorzugt aus Propen), umfassend die Umsetzung der Verbindung, die eine Kohlenstoff-Kohlenstoff-Doppelbindung enthält, mit Wasserstoffperoxid in Gegenwart des erfindungsgemäßen Katalysators.

## Beschreibung

Die vorliegende Erfmdung betrifft einen Katalysator enthaltend Titan in gebundener Form, nichtkristallines Siliziumdioxid und mindestens eine kristalline Silikatphase, die eine Zeolithstruktur aufweist, wobei das nichtkristalline Siliziumdioxid auf mindestens einer der kristallinen Silikatphasen, die eine Zeolithstruktur aufweisen, aufgebracht ist, und wobei mindestens eine der kristallinen Silikatphaseri, die eine Zeolithstruktur aufweisen, Silizium-Kohlenstoff-Bindungen enthält, mit denen nicht hydrolytisch abtrennbare organische Gruppen R an Silizium gebunden sind. Weiterhin betrifft die vorliegende Erfindung ein Verfahren zur Herstellung dieses Katalysators und ein Verfahren zur Herstellung eines Epoxides aus einer Verbindung, die eine Kohlenstoff-Kohlenstoff-Doppelbindung enthält (bevorzugt aus Propen), umfassend die Umsetzung der Verbindung, die eine Kohlenstoff-Kohlenstoff-Doppelbindung enthält, mit Wasserstoffperoxid in Gegenwart des erfindungsgemäßen Katalysators.

Aus EP-A 0 100 119, EP-A 1 221 442, DE-A 199 54 322 und aus EP-A 0 904 151 ist bekannt, dass sich Olefine mit Wasserstoffperoxid zu einem Epoxid umsetzen lassen, wenn als Katalysator ein rein anorganischer titanhaltiger Zeolith eingesetzt wird.

Alle diese offenbarten Katalysatoren weisen jedoch den Nachteil auf, dass sich die verwendeten Oxidationsmittel (Wasserstoffperoxid; Ethyl- bzw. Isopropylbenzolhydroperoxid) an diesen Katalysatoren partiell zersetzen. Die Folgen sind Epoxidausbeuten bezogen auf das Oxidationsmittel von <100 % und in einigen Fällen sicherheitstechnische Probleme durch Bildung von molekularem Sauerstoff als ein Zersetzungsprodukt des Oxidationsmittels.

Weiterhin weisen alle offenbarten Katalysatoren den Nachteil auf, dass sie während der Reaktion zunehmend an katalytischer Aktivität verlieren.

Die Lehre von WO 99/01445 hält den gewünschten Olefin-Mindestumsatz für eine begrenzte Zeit durch Erhöhung von Reaktionstemperatur und/oder -druck konstant. Die technischen Grenzen sind aufgrund der hohen Epoxid-Reaktionsfähigkeit allerdings sehr eng. Schon geringe Temperaturerhöhungen können die Epoxid-Selektivität merklich verkleinern. Bei technischen Anlagen im Kilo-Tonnenmaßstab können kleine Verringerungen der Produktselektivitäten die Wirtschaftlichkeit gefährden.

EP-A 0 743 094 und EP-A 0 790 075 beschreiben die thermische Regenerierung bevorzugt mit molekularem Sauerstoff. Zur Erreichung der Zieltemperaturen von 200, besser 550°C muss der Katalysator in einigen Fällen aus dem Reaktor ausgebaut werden. Zumindest ist den offenbarten Regenerierungen gemeinsam, dass die Epoxidationsreaktion für den Zeitraum der Regenerierung unterbrochen werden muss.

Kurze Katalysatorstandzeiten bedingen Produktionsausfall während der Regenerierungsphase oder verlangen nach einem redundanten, kostenintensiven Produktionsweg. Es ist also eine Entwicklung von neuen Katalysatoren wünschenswert, die bei hohen Selektivitäten hohe Aktivitäten bei technisch interessanten Standzeiten erreichen können.

EP-A 1 221 442 beschreibt die Regenerierung des Titanzeolithkatalysators TS1 mit wässrigem Wasserstoffperoxid. Die Offenlegung zeichnet sich besonders dadurch aus, dass die Regenerierung während der in einem kontinuierlich durchströmten Reaktionssystem erfolgten Epoxidierungsreaktion, also in Gegenwart von Olefin, Methanol und wässrigem Wasserstoffperoxid, durchgeführt werden kann.

Der Mechanismus der Deaktivierung ist nicht vollständig geklärt. Vermutlich erfolgt eine Belegung der katalytisch aktiven Feststoffoberfläche mit organischen Molekülen in einem solchen Ausmaße, dass die aktiven Epoxidierungsspezies nicht mehr für die gewünschte Reaktion zur Verfügung stehen.

DE-A 199 54 322 beschreibt TS1-Formkörperkatalysatoren, die sich dadurch auszeichnen, dass sich die Extrudate aus TS1-Pulver und anderen Materialien auf SiO₂-Basis zur Formgebung zusammensetzen. Diese Extrudate, bestehend also aus kristalliner und nicht-kristalliner SiO₂-Phase werden mit Aminopropyltrialkoxysilan imprägniert, einer Base und gleichzeitig einem Reagenz zur Modifizierung der Extrudatoberfläche, und nachfolgend bei 550 °C im Luftstrom so lange kalziniert, bis keine Silizium-Kohlenstoffbindung analytisch mehr nachweisbar ist. Die so erhaltenen rein anorganischen Formkörperkatalysatoren haben zwar die gleiche katalytische Aktivität wie analoge Systeme ohne Silanoberflächenbehandlung in einem der TS1-Synthese nachgeschalteten Reaktionsschritt, zeigen aber die Tendenz, geringfügig weniger Nebenprodukte zu generieren. Zusätzlich liegt die Seitendruckstabilität der Extrudat-Stränge um 50 % höher. Die Folgereaktion von PO mit Wasser bzw. Methanol zu Propylenglykol und Methoxypropanol wird an den offenbarten Katalysatoren offensichtlich ein wenig zurückgedrängt.

Dies zeigen die Daten in folgender Tabelle:

| | ohne Modifizierung | mit Modifizierung |
|---|---|---|
| - Methoxypropanol [ppm]: | 3100-3800 | 1700-3200 |
| - Propandiol [ppm] | 400-600 | 300-500 |

Für einen technischen Prozess ist eine Entwicklung von Katalysatoren wünschenswert, die bei noch höheren Epoxid-Selektivitäten und Epoxid-Produktivitäten längere Katalysatorstandzeiten erreichen. Weiterhin wäre es wünschenswert, weniger das teure Oxidationsmittel durch Zersetzung am Katalysator und zur Katalysatorregenerierung zu vergeuden.

Zur Herstellung von Katalysatoren im technischen Maßstab (Tonnen-Maßstab) sollten die Verfahrensschritte zur Katalysatorpräparation möglichst reproduzierbar und einfach sein. Zur Realisierung eines wirtschaftlichen Verfahrens sollen die Kosten zur Katalysatorherstellung sehr niedrig sein.

Eine Aufgabe der vorliegenden Erfmdung bestand also darin, neue Katalysatoren für technische Prozesse mit hohen Aktivitäten ohne Deaktivierung bei gleichzeitig exzellenten Selektivitäten und möglichst wenig Verlust von Oxidationsmittel durch Zersetzung am Katalysator bereitzustellen.

Eine weitere Aufgabe bestand darin, ein Verfahren zur Herstellung dieser Katalysatoren zu entwickeln.

Eine weitere Aufgabe bestand darin, ein technologisch einfaches Flüssigphasenverfahren zur selektiven Oxidation von Kohlenwasserstoffen mit Wasserstoffperoxid an diesen Katalysatoren zur Verfügung zu stellen, welches bei hohen Aktivitäten, sehr hohen Selektivitäten und technisch interessanten Katalysatorstandzeiten zu hohen Ausbeuten und geringen Kosten führt.

Eine weitere Aufgabe bestand darin, einen alternativen Katalysator zur Direktoxidation von Kohlenwasserstoffen bereitzustellen.

Eine weitere Aufgabe bestand darin, die Nachteile der bekannten Katalysatoren wenigstens teilweise zu beseitigen.

Diese Aufgaben werden gelöst durch den Katalysator, das Verfahren zu seiner Herstellung und das Verfahren zur Herstellung eines Epoxides gemäß den Hauptansprüchen der vorliegenden Schrift.

Die kristalline Silikatphase, die eine Zeolithstruktur aufweist und die Silizium-Kohlenstoff-Bindungen enthält, mit denen nicht hydrolytisch abtrennbare organische Gruppen R an Silizium gebunden sind, wird organisch-anorganischer Hybrid-Zeolith genannt.

Besondere Ausführungsformen der vorliegenden Erfindung sind in den Unteransprüchen beschrieben.

Die organischen Gruppen R sind bevorzugt in einer Menge von 0,01 bis 5 Gew.-%, besonders bevorzugt 0,1 bis 4 Gew.-% und ganz besonders bevorzugt 0,3 bis 2 Gew.-%, vorhanden, bezogen auf die Menge der kristallinen Silikatphasen, die eine Zeolithstruktur aufweisen und die Silizium-Kohlenstoff-Bindungen enthalten, mit denen nicht hydrolytisch abtrennbare organische Gruppen R an Silizium gebunden sind.

Bei der Herstellung des erfindungsgemäßen Katalysators erfolgt das Kalzinieren bei einer Temperatur von 100 bis 550°C, bevorzugt bei 200 bis 450°C.

Die vorliegende Erfindung betrifft also insbesondere Zusammensetzungen enthaltend hauptsächlich die Elemente Silizium, Titan, Sauerstoff und Kohlenstoff, wobei diese Zusammensetzungen neben den rein anorganischen Bestandteilen auch organische Silizium-Kohlenstoffbindungen aufweisen, und die Zusammensetzungen weiterhin mindestens eine kristalline Phase im Oxid des Siliziumoxids mit Zeolithstruktur enthalten. Die erfindungsgemäßen kristallinen Systeme, die sowohl anorganische, als auch organische Bestandteile homogen verteilt enthalten, werden in der vorliegenden Schrift als Hybridsysteme bezeichnet. Die Erfindung offenbart sowohl die Synthese dieser Hybrid-Zusammensetzungen, als auch die Verwendung dieser Hybrid-Zusammensetzungen als Katalysator.

Zeolithe sind kristalline, mikroporöse Alumosilikate, deren Kristallgitter aus SiO₄- und AlO₄-Tetraeder aufgebaut sind. Dieser Aufbau führt zu streng regelmäßig geformten Hohlräumen oder Kanälen, deren Abmessungen in derselben Größenordnung (0,3-1,5 nm) liegen, wie die Abmessungen von zahlreichen Molekülen.

Die Verwendung Al-reicher Zeolithe (A, X oder Y) als hydrophile Adsorbentien beruht auf der hohen Polarität, die durch die Anwesenheit von Al im Gitter erzeugt wird. Eine Senkung des Al-Gehaltes, durch Dealuminierung oder, wenn möglich, durch entsprechende Syntheseansätze, führt zu einer Erniedrigung der Polarität des Gitters und somit zu einem zunehmend hydrophoben Charakters der Adsorbentien. Führt man Synthesen des Zeoliths ZSM-5 bei Abwesenheit von Al durch, erhält man Silicalit-1, eine Modifikation des SiO₂ und ein typisch hydrophobes Adsorbens.

Der Einbau von Ti in das Gitter von Silicalit-1 erhöht wiederum dessen Polarität. Dies führt zur Zunahme der Adsorptionskapazität für Wasser und zu einer Abnahme der Kapazität für unpolare Adsorptive (S. Mirajkar et al., J. Phys. Chem. 96, 3073/3079 (1992)). Die Hydrophobizität sollte also mit zunehmendem Ti-Gehalt abnehmen. Außerdem sollte man unterscheiden können, ab das Ti im Gitter eingebaut oder als amorphes TiO₂ vorliegt, da das letztgenannte die Polarität des Gitters nicht beeinflusst.

Die im TS1 tetraedrisch eingebauten Ti-Atome (bevorzugt ca. 1,3 Mol-%) stellen die sogenannten actives sites dar. Aus katalytischer Sicht wäre es einerseits wünschenswert, mehr Ti-Spezies ohne Bildung von amorphen TiO₂ (als Niederschlag) in das Silicalitgitter einzubauen, andererseits sollte ein möglichst hydrophobes Katalysatorsystem (wird durch möglichst geringen Ti-Anteil erreicht) die Propen-Adsorption sowie vor allem die PO-Desorption deutlich steigern und somit PO-Folgereaktionen mit Wasser zu Glykolen an active sites reduzieren.

Überraschend ist mit der vorliegenden Erfmdung die Synthese eines Titansilikaliten gelungen, der sich dadurch auszeichnet, dass nicht-hydrolysierbare organische Liganden in das xTiO₂(1-x)SiO₂ Netzwerk homogen so eingebaut sind, dass die stabile kristalline Struktur mit ihren regelmäßig geformten Hohlräumen und Kanälen überwiegend erhalten bleibt. Der homogene Einbau der nicht-hydrolysierbaren organischen Liganden gelingt bevorzugt, indem Cokondensationsmittel mit unpolaren Kohlenwasserstoffen in das Polymer integriert werden.

Die erfindungsgemäßen Katalysatoren werden in der vorliegenden Schrift als Hybrid-Titansilikalite oder auch Hybrid-TS1 bezeichnet. Die Kristallinität ist insofern für die erfindungsgemäßen Katalysatoren bevorzugt, weil die tetraedrischen Titanzentren insbesondere in der Stabilität eines kristallinen Gitters unter den Epoxidierungsbedingungen stabil und katalytisch aktiv bleiben.

Überraschend ist weiterhin, dass die organischen Liganden den Formierungsprozess von der amorphen Anordnung zur kristallinen ZSM-5 Struktur nur wenig behindern.

Die erfindungsgemäßen Katalysatoren zeichnen sich besonders dadurch aus, dass die Zersetzung von Wasserstoffperoxid an diesen Systemen deutlich verringert wird. In vielen Fällen steigt die Stabilität von H₂O₂ in Gegenwart von den Hybrid-TS1 um mehr als den Faktor 1,5-2 im Vergleich zu konventionellem rein anorganischem TS1.

Die erfindungsgemäßen Hybrid-Systeme mit erhöhter Hydrophobizität sind für Epoxidationsreaktionen mit Wasserstoffperoxid besonders wertvoll, weil das erfindungsgemäße Hybrid-TS1 mit seiner verringerten Polarität sich offenbar gezielt an die Bedürfnisse der katalytischen Reaktion genau anpasst. Experimentell konnte eindeutig nachgewiesen werden, dass die Diffusionsprobleme von den unpolaren Edukten (z.B. Olefine wie Propen) und den polaren Produkten (z.B. PO) deutlich minimiert werden. Der hydrophobe Charakter verleiht dem Material zusätzlich Stabilität gegenüber Wasserdampf, welche die Katalysatorlebensdauer weiter verlängert.

Die TS1 Synthese, erstmalig 1983 von Clerico (ENICHEM) veröffentlicht, ist hinreichend bekannt.

Die erfindungsgemäßen Hybrid-TS1 Synthesen erfolgten sowohl in Anlehnung an die Einstufensynthese nach EP-B 0 904 151, als auch in Anlehnung nach der Zwei-Schritt-Synthese nach P. Serrano/M.A. Uguina/R. von Grieken/ M. Camacho, Appl. Catal., A 1995, 124(2), 391-408.

Bei der klassischen Ein-Stufen-Synthese dient das Templatmolekül (Tetrapropylammoniumhydroxid als Schablone) sowohl zur Hydrolyse/Kondensation der Silizium- und Titanprecursoren, als auch zur ZSM-5-Zeolithstrukturbildung.

Die Zwei-Schritt Synthese, auch als Imprägniermethode zur Synthese von rein anorganischem TS1 bekannt, teilt sich in folgende zwei Schritte auf:
- Schritt 1:: Herstellung eines amorphen SiO₂-TiO₂-Sol-Gel-Zwischenproduktes (Cogel) aus Silizium- und Titanalkoxiden (stabiler Einbau von Ti(IV)-O-Si-Spezies vor der eigentlichen Zeolithsynthese (Heterokondensation)).
- Schritt 2:: Überführung des amorphen Cogels in eine Zeolithstruktur durch Imprägnierung mit einem Templat (Tetrapropylammoniumhydroxid) und anschließender Hydrothermalsynthese (Autoklavenreaktion).

Im folgenden wird die Hybrid-TS1-Katalysator beschrieben.

Die organisch-anorganischen Hybridmaterialien im Sinne der Erfindung enthalten bevorzugt mindestens eine kristalline Phase auf Hybrid SiO₂/RSiOₓ-Basis.

Bezogen auf kristallines Siliziumdioxid, der Hauptbestandteil der erfindungsgemäßen Materialien, enthalten die Hybrid-Systeme bevorzugt zwischen 0,1 und 4 Mol-% Titan, bevorzugt zwischen 0,5 und 2 Mol-%, besonders bevorzugt zwischen 0,8 und 1,6 Mol-%.

Das Titan liegt bevorzugt in oxidischer Form vor und ist bevorzugt chemisch über Si-O-Ti-Bindungen in das organisch-anorganische Hybridmaterial eingebaut oder angebunden. Aktive Katalysatoren dieser Art weisen nur sehr untergeordnet Ti-O-Ti-Domänen auf.

Bevorzugt ist, dass in aktiven Katalysatoren Titan über Heterosiloxanbindungen an Silizium gebunden ist.

Bezogen auf kristallines Siliziumdioxid als Basiskomponente der Wirksubstanz, enthalten die Hybrid-Systeme bevorzugt zwischen 0,01 und 5 Mol-% nicht-hydrolysierbare organische Liganden, bevorzugt zwischen 0,05 und 4 Mol-%, besonders bevorzugt zwischen 0,2 und 1,5 Mol-%. Die nicht-hydrolysierbaren organischen Liganden sind bevorzugt homogen in das organisch-anorganische Hybridmaterial eingebaut oder angebunden.

Die erfindungsgemäßen homogenen Hybrid-Zusammensetzungen, enthaltend Silizium- Titan- und Kohlenstoffatome, können in einer besonderen Ausführungsform im getrockneten Zustand näherungsweise durch folgende Formel (I) beschrieben werden (die nach Modifizierung gebildeten Reste an der Oberfläche und gegebenenfalls unvollständig abreagierte Gruppen werden hier nicht berücksichtigt):

(TiO₂)ₓ(SiO₂)₍₁₋ₓ₎/ (TiO₂)_{y}(RSiO_{1,5})_{(1-y)}/M (I)

(TiO₂)ₓ(SiO₂)₍₁₋ₓ₎ steht in dieser Formel (I) für rein anorganisches kristallines TS1 (MFI-Kristallstruktur) oder TS2 (MEL-Kristallstruktur), (TiO₂)_{y}(RSiO_{1,5})_{(1-y)} steht für organisch modifiziertes TS 1 (Hybrid-TS1; durch den Hybrid-TS1-Anteil wird der gesamte Molekülverbund - bestehend aus xTiO₂(1-x)SiO₂ / xTiO₂(1-x)RySiO_{4-y} - in der vorliegenden Schrift als Hybrid-TS1 bezeichnet).

M ist in Formel (I) ein Heteroatom, welches zusätzlich zum Titan, bevorzugt Sn, Fe, Al, Ge oder Kombinationen davon, in den Molekülverband eingebracht werden kann.

x und y stehen in Formel (I) für die effektiv notwendige Anzahl an Sauerstoffatomen um die Valenzen von Si und Ti abzusättigen.

Die oben bezeichnete Zusammensetzung (I) lässt sich über weite Bereiche variieren.

Die spezifische Oberfläche der organisch-anorganische Hybridmaterialien ist nicht eingeschränkt. Die spezifische Oberfläche der Katalysatoren in den angehängten Beispiele liegt im Bereich 0,5-100 m²/g. Systeme mit kleineren oder größeren Oberflächen sind aber ebenfalls katalytisch aktiv.

Geeignete Vorläuferverbindungen für Silizium-, Titan- und Promotorzentren sind vorteilhaft entsprechende für den Sol-Gel-Prozess geeignete niedermolekulare organisch-anorganische Mischverbindungen oder eine Kombination aus entsprechenden anorganischen und organisch-anorganischen Mischverbindungen. Niedermolekular steht im Sinne der Erfindung für monomer oder oligomer. Bei ausreichender Löslichkeit sind auch polymere Vorläuferverbindungen von Silizium, Titan und Promotoren geeignet.

Bevorzugte Lösungsmittel für den Sol-Gel-Prozeß sind Alkohole wie Isopropanol, Butanol, Ethanol, Methanol oder Ketone wie Aceton, und Ether wie z.B. THF oder tert.-Butylmethylether.

Geeignete Ausgangsmaterialien sind insbesondere sämtliche dem Fachmann bekannten löslichen Silizium- und Titanverbindungen der allgemeinen Formel (II), die als Ausgangsmaterial für die entsprechenden Oxide oder Hydroxide dienen können,

[RₓM'(OR')₄₋ₓ] (II),

wobei
- M': ausgewählt wird aus Silizium und Titan,
- R und R': gleich oder verschieden sind und unabhängig voneinander ausgewählt werden aus der Gruppe C₁-C₁₂-Alkyl, und C₆-C₁₂-Aryl,
wobei
- x: = 0,1,2,3 ist und
- R': auch H sein kann.

Bei den organisch modifizierten Silanen sind in einer besonderen Ausführungsform eine oder mehrerer hydrolysierbarer Gruppen durch terminale und/oder verbrückte gesättigte (z. B. CH₃, C₂H₅, C₃H₇, ..) oder durch ungesättigte (z. B. C₂H₃, C₆H₅) R-Gruppe(n) ersetzt worden. Es können auch polyfunktionelle Organosilane, z. B. Silanole und Alkoxide, eingesetzt werden. Es können auch Silane, organisch modifiziert oder auch nicht, in Gegenwart von Di- oder Mehrfachalkoholen wie 1,4-Butandiol, zu organisch modifizierten Polysiloxanen umgesetzt werden. Verbrückte R-Gruppen (Alkylenreste) sind im Sinne der Erfindung verbrückte Strukturen wie kettenförmige, sternförmige (verzweigt), käfigförmige oder ringförmige Strukturelemente.

Für die Synthese der erfindungsgemäßen Katalysatoren werden organisch modifizierte Siliziumprecursor bevorzugt, bei denen der sterische Anspruch des organischen Liganden relativ gering ist, wie z.B. Methyltrimetoxysilan, Methyltriethoxysilan, Methyltrialkoxysilan, Ethyltrimethoxysilan, Ethyltriethoxysilan oder ähnliche Precursor.

Anstelle monomerer Alkoxide können auch deren Kondensationsprodukte verwendet werden.

Die erfindungsgemäßen Zusammensetzungen können in jeder beliebigen physikalischen Form für Oxidationsreaktionen eingesetzt werden, z.B. gemahlene Pulver, spherische Partikel, Pellets, Extrudate, Granulate.

In einer bevorzugten Ausführungsform werden die pulverförmigen kristallinen Hybrid-TS1 Systeme in mechanisch stabile Formkörper überführt. Formkörper sind die bevorzugte Modifikation zur Befüllung von Festbettreaktoren, wie z.B. Rohrreaktoren. Formkörper durch verfestigende Formgebungsprozesse sind hinreichend bekannt, wie z.B. Strangextrudierung (z.B. Stränge mit 1-12 mm Durchmesser) nach EP-B 0 904151 oder DE-A 199 54 322).

Den erfindungsgemäßen Hybrid-TS1 Systemen werden zur Strangextrudierung vorteilhaft bis zu 15 Gew.-% Bindemittel (bezogen auf die Gesamtmasse des kalzinierten bzw. getemperten Katalysators) beigemengt. Siliziumdioxid, amorph oder kristallin, in feinen Pulvern, oder als Si-Vorläuferverbindung (z.B. Tetraethoxysilan) wird als unpolares Bindemittel bevorzugt. Andere Bindemittel, wie z.B. in EP-B 0 904 151 beschrieben, sind dann geeignet, wenn sie die Lewis-Accidität des Formkörpers nicht deutlich erhöhen (Lewis-Zentren können mit funktionellen organischen Gruppen, z.B. Epoxidfunktionen, reagieren und auf diese Weise Nebenprodukte erzeugen).

Zum Anteigen einer Mischung aus dem erfindungsgemäßen Material, dem Bindemittel und Lösungsmittel, wie z.B. Wasser, Alkohol oder Mischungen davon, können Hilfsstoffe wie Methylcellulose - wie aus der Literatur hinreichend bekannt - in kleinen Mengen eingesetzt werden.

Der Anteil der Hilfsstoffe, bezogen auf die Gesamtmasse, sollte bei den erfindungsgemäßen Materialien bevorzugt unter 5 Gew.-%, besonders bevorzugt unter 2,5 Gew.-% liegen.

Im folgenden werden die Titanzentren beschrieben.

Precursoren für die Titanzentren sind nicht festgelegt. Beispielsweise können Titanalkoxide, lösliche Titansalze, oder titanorganische Verbindungen verwendet werden.

Obwohl alle Salze von Titan, wie Halogenide, Nitrate und Hydroxide verwendet werden können, werden Titanalkoxide, z.B. Butoxid, Isopropoxid, Propoxid, Ethoxid bevorzugt.

Bevorzugt werden Titanderivate wie Tetralkoxytitanate, mit Alkylgruppen von C₁-C₁₀ wie iso-Butyl, tert-Butyl, n-Butyl, i-Propyl, n-Propyl, Ethyl, usw., oder Titanalkoxykomplexe wie in US-A 6 090 961 beschrieben, z.B. ((η5-tetramethylcyclopentadienyl)3-tert-butyl-5-methyl-2-phenoxy)-dimethylsilyl-titanium-dimethoxide oder andere organische Titanspezies wie Titanylacetylacetonat, Ti(OsiPh₃)₄, Dicyclopentadienyltitandihalogenid, Titandihalogendialkoxid, Titanhalogentrialkoxid, Titaniumsiloxane wie z. B. Diethoxysiloxane-ethyl-titanat copolymer (kommerziell bei der Firma Gelest Inc. erhältlich), verwendet. Bei Halogensubstituenten wird Chlor bevorzugt. Es können auch Mischalkoxide von Titan mit anderen Elementen wie z.B. Titantriisopropoxid-tri-nbutylstannoxid eingesetzt werden. Die Titanvorläuferverbindungen können auch in Gegenwart von komplexbildenden Komponenten wie z. B. Acetylaceton oder Ethylacetoacetat eingesetzt werden.

Zur Synthese der erfindungsgemäßen Zusammensetzungen werden Tetralkoxytitanate als Titanprecusoren, wie z.B., mit Alkylgruppen von C₂-C₆ wie iso-Butyl, tert-Butyl, n-Butyl, i-Propyl, n-Propyl, Ethyl, bevorzugt.

Im folgenden wird die thermische Aktivierung beschrieben.

Die erfindungsgemäßen Zusammensetzungen werden bevorzugt nach der Hydrothermalsynthese durch thermische Behandlung bei 100-500°C in verschiedenen Atmosphären wie Luft, Stickstoff, Wasserstoff weiter aktiviert. Bevorzugt wird das erfindungsgemäße kristalline Material bei 80-120°C getrocknet und anschließend unter Inertgas auf 300-500°C erwärmt. In einigen Fällen kann vorteilhaft sein, die thermische Aktivierung bei der Zieltemperatur - 300-500°C in sauerstoffhaltiger Atmosphäre zu vervollständigen. Die Kalziniertemperatur und -dauer richtet sich nach dem Ziel-Gehalt an organischen Liganden im erfindungsgemäßen System. Ab 450°C setzt der Abbrand der organischen Spezies, besonders in sauerstoffhaltiger Atmosphäre, merklich ein.

Die thermisch aktivierten (getemperten) erfindungsgemäßen Hybrid-Zusammensetzungen zeigen häufig eine signifikant höhere katalytische Aktivität bei der Epoxidation mit Wasserstoffperoxid und eine verlängerte Katalysatorstandzeit im Vergleich zu bekannten rein anorganischen TS1-Katalysatoren.

Die erfindungsgemäßen Zusammensetzungen deaktivieren langsam mit der Zeit.

Für rein anorganische TS1-Systeme ist die Regenerierung durch Waschung mit Wasserstoffperoxidlösung bekannt (zum Beispiel aus EP-A 1 221 442).

Überraschend wurde gefunden, dass sich die erfindungsgemäßen Hydridsysteme trotz ihrem Anteil an homogen ein- bzw. angebauter Organik durch Waschen mit Wasserstoffperoxidlösungen (z.B. 3 bis 40 %-ige H₂O₂-Methanol-Lösusung) vollständig regenerieren lassen. Dieser Befund ist insofern überraschend, weil die organischen Liganden theoretisch auch von dem Oxidationsmittel Wasserstoffperoxid aufoxidiert werden könnten. Ein Dauertestversuch von 500 h zur Epoxidation mit Hydrid-TS1/Regenerierung von Hydrid-TS1 im kg-Maßstab zeigte keinerlei Verlust der organischen Liganden (IR-Analyse der Hybrid-TS1-Zusammensetzungen; Pulver und Formkörper).

Die erfindungsgemäße Zusammensetzung kann grundsätzlich auf alle Kohlenwasserstoffe angewendet werden. Unter dem Begriff Kohlenwasserstoff werden ungesättigte oder gesättigte Kohlenwasserstoffe wie Olefine oder Alkane verstanden, die auch Heteroatome wie N, O, P, S oder Halogene enthalten können. Die zu oxidierende organische Komponente kann azyklisch, monozyklisch, bizyklisch oder polyzyklisch und kann monoolefinisch, diolefinisch oder polyolefinisch sein. Bei organischen Komponenten mit zwei oder mehreren Doppelbindungen können die Doppelbindungen konjugiert und nichtkonjugiert vorliegen.

Bevorzugt sind ungesättigte Kohlenwasserstoffe mit 2 bis 15, vorzugsweise 2 bis 10 Kohlenstoffatomen, insbesondere Ethen, Propen, Isobutylen, 1-Buten, 2-Buten, cis-2-Buten, trans-2-Buten, 1,3-Butadien, Pentene, 1-Hexen, andere Hexene, Hexadien, Cyclohexen, Benzol.

Im folgenden werden Prozessparameter beschrieben.

Hybrid-TS1-Katalysatoren werden bevorzugt in Flüssighasenreaktionen zur Partialoxidation von Kohlenwasserstoffen in Gegenwart von Wasserstoffperoxid eingesetzt. Hybrid-TS1-Katalysatoren sind ebenfalls in der Gasphase aktiv.

Die Prozessparameter für die Hydro-Oxidationsreaktion in der Flüssigphase können in weiten Teilen variiert werden.

Die erfindungsgemäßen HO-Katalysatoren arbeiten insbesondere bei Temperaturen von 30 bis 200°C, bevorzugt bei 40 bis 80°C und besonders bevorzugt bei 40 bis 70°C.

Für Flüssigphasenreaktionen ist es aus wirtschaftlichen und Apparatebaugründen oft vorteilhaft, unter erhöhten Reaktionsdrücken zu arbeiten. Die erfindungsgemäßen Kontakte zeigen insbesondere hohe katalytische Aktivitäten im Druckbereich von Normaldruck bis 70 bar. Bevorzugt wird ein Druckniveau von 2 bis 35 bar, besonders bevorzugt von 5 bis 30 bar.

Die Verweilzeit kann ebenfalls in weiten Bereichen variiert werden. Vorteilhaft liegt die Verweilzeit bei <70 Sekunden. Die Hybrid-TS1-Formkörperkatalysatoren zeigen besonders hohe katalytische Aktivitäten mit guten Selektivitäten bei Verweilzeiten <90 sec. Sehr kurze Verweilzeiten im unteren Sekundenbereich (< 40 Sekunden) sind auch Gegenstand der vorliegenden Erfindung.

Im Folgenden wird die Feed-Zusammensetzung beschrieben.

Hybrid-TS1-Katalysatoren werden bevorzugt in Flüssigphasenreaktionen zur Partialoxidation von Kohlenwasserstoffen in Gegenwart von Wasserstoffperoxid eingesetzt.

Hierbei werden bevorzugt selektiv aus Olefinen Epoxide, aus gesättigten sekundären Kohlenwasserstoffen Ketone und aus gesättigten tertiären Kohlenwasserstoffen Alkohole erhalten.

Die molare Menge des eingesetzten Kohlenwasserstoffs in Bezug auf die Gesamtmolzahl aus Kohlenwasserstoff, Verdünnungsgas, Wasserstoffperoxid und Lösungsmittel sowie das relative molare Verhältnis der Komponenten kann in weiten Bereichen variiert werden. Bevorzugt wird ein Überschuss von Kohlenwasserstoff, bezogen auf eingesetzten Sauerstoff (auf molarer Basis) eingesetzt. Der Kohlenwasserstoffgehalt liegt typischerweise größer 1 Mol-% und kleiner als 80 Mol-%. Bevorzugt werden Kohlenwasserstoffgehalte im Bereich von 4 bis 90 Mol-%, besonders bevorzugt im Bereich von 8 bis 70 Mol-% eingesetzt.

Der Sauerstoff kann in verschiedenster Form eingesetzt werden, wie molekularer Sauerstoff, Luft, Stickstoffoxid, Wasserstoffperoxid. Molekularer Sauerstoff wird bevorzugt.

Der molare Sauerstoffanteil, in Bezug auf die Gesamtmolzahl aus Kohlenwasserstoff, Sauerstoff, Wasserstoff und Verdünnungsgas, kann in weiten Bereichen variiert werden. Bevorzugt wird der Sauerstoff im molaren Unterschuss zum Kohlenwasserstoff eingesetzt. Bevorzugt werden im Bereich von 1-30 Vol.%, besonders bevorzugt 5-25 Vol.% Sauerstoff eingesetzt.

In Abwesenheit von Wasserstoff zeigen die erfindungsgemäßen Formkörper nur geringe Aktivität und Selektivität. Bis 180°C ist in der Regel die Produktivität in Abwesenheit von Wasserstoff gering, bei Temperaturen größer 200°C werden neben Partialoxidationsprodukten größere Mengen Kohlendioxid gebildet.

Es kann jede bekannte Wasserstoffquelle genutzt werden, wie reiner Wasserstoff, Cracker-Wasserstoff, Synthesegas oder Wasserstoff aus Dehydrierung von Kohlenwasserstoffen und Alkoholen. In einer Ausführungsform der Erfindung kann der Wasserstoff auch in einem vorgeschalteten Reaktor in situ erzeugt werden, z.B. durch Dehydrierung von Propan oder Isobutan oder Alkoholen wie z.B. Methanol oder Isobutanol. Der Wasserstoff kann auch als Komplexgebundene Spezies, z.B. Katalysator-Wasserstoffkomplex, in das Reaktionssystem eingeführt werden.

Der volumetrische Anteil an Wasserstoffperoxid, in Bezug auf das Gesamtvolumen, bestehend hauptsächlich aus den Komponenten Methanol/Wasser/Wasserstoffperoxid/Kohlenwasserstoff, kann in weiten Bereichen variiert werden. Typische Wasserstoffperoxidgehalte liegen bei 10-40 Vol.-%, bevorzugt 15-40 Vol.-%, besonders bevorzugt bei 17-30 Vol.-%.

Zu den essentiell notwendigen oben beschriebenen Eduktgasen kann optional auch ein Verdünnungsgas, wie Stickstoff, Helium, Argon, Methan, Kohlendioxid, Kohlenmonoxid oder ähnliche, sich überwiegend inert verhaltende Gase, eingesetzt werden. Auch Mischungen der beschriebenen Inertkomponenten können eingesetzt werden. Als Feed- bzw. kreisgasverdünnende Komponenten können auch andere inerte Kohlenwasserstoffe eingesetzt werden, wie beispielsweise fluorierte Kohlenwasserstoffe (Hexafluorethan, CF4, und andere). Der Inertkomponentenzusatz ist zum Transport der freiwerdenden Wärme dieser exothermen Oxidationsreaktion und häufig aus sicherheitstechnischen Gesichtspunkten günstig.

Die erfindungsgemäßen HO-Katalysatoren weisen einen hohen wirtschaftlichen Vorteil gegenüber dem Stand der Technik auf. Weiterhin zeigen die erfindungsgemäßen Systeme eine deutlich längere Katalysatorlebensdauer als herkömmliche rein anorganische Titansilikalith-Katalysatoren.

Die erfindungsgemäßen Katalysatoren lassen sich verfahrenstechnisch problemlos und kostengünstig im technischen Maßstab herstellen.

Die vorliegende Erfindung wird durch die folgenden Beispiele erläutert. Die vorliegende Erfindung ist nicht auf diese Beispiele beschränkt.

### Beispiele

### Vorschrift zum Test der HO-Formkörperkatalysatoren (Testvorschrift)

Es wurde ein 250 ml Glasautoklav der Firma Büchi im Semibatch-Betrieb eingesetzt, welcher mittels eines Thermostaten (Öl) temperiert wurde. Der Reaktor wurde mit einem Satz von zwei Massendurchflussregler (Propen, Stickstoff) kontinuierlich mit Feedgasen versorgt. Zur Reaktion wurden 0,2 g Hybrid-TS1 Pulverkatalysatoren - suspendiert in einem Methanol/Wasser Gemisch (15 g Methanol, 5 g 30 %ige Wasserstoffperoxidlösung in Wasser) - bei 50°C und 3 bar vorgelegt. Ein Druckhalteventil der Firma Swagelok sorgt für den konstanten Druck. Die Suspension wurde mittels eines Magnetrührkerns mit 800 U/min⁻¹ gerührt. Die Eduktgase werden durch eine 1 mm 0,2 mm Kapillare direkt in die Suspension eindosiert (Tauchung). Die Standardwirksubstanzbelastung lag bei 21 1 Gas/(g TS1 * h). Zur Durchführung der Oxidationsreaktionen mit 0,2 g TS1 wurde ein Gasstrom, nachfolgend immer als Standard-Gaszusammensetzung bezeichnet, ausgewählt: 0,252 l/h C₃H₆, 3,96 l/h N₂ (entspricht 6 % Propen in N₂).

In den nachfolgenden orientierenden Versuchen wurde der Einfachheit halber nur die PO-Vol.-Konzentration im austretenden Gasstrom quantitativ via GC-Analyse dedektiert; die in der Flüssigphase gelöste PO-Menge wurde zur Auswertung qualitativ berücksichtigt. (PO ist die Abkürzung für Propylenoxid).

Die Reaktionsgase wurden gaschromatographisch quantitativ analysiert. Die gaschromatographische Auftrennung der einzelnen Reaktionsprodukte erfolgte durch eine kombinierte FID/WLD-Methode, bei der drei Kapillarsäulen durchlaufen werden.

| | |
|---|---|
| FID: | HP-Innowax, 0,32 mm Innendurchmesser, 60 m lang, 0,25 µm Schichtdicke. |
| WLD: | Hintereinanderschaltung von |
| HP-Plot Q, 0,32 mm Innendurchmesser, 30 m lang, 20 µm Schichtdicke | |
| HP-Plot Molsieve 5 A, 0,32 mm Innendurchmesser, 30 m lang, 12 µm Schichtdicke. | |

Die Abkürzungen bedeuten folgendes:

| | |
|---|---|
| FID | Flammenionisationsdetektor |
| WLD | Wärmeleitfähigkeitsdetektor |
| HP-Plot Q | Gaschromatographiesäule der Firma Hewlet Packard (fused silica; PLOT = porous layer open tubular) |
| HP-Plot Molsieve 5 A | Gaschromatographiesäule der Firma Hewlet Packard (Molsieb 5 Angström; PLOT = porous layer open tubular) |

### Beispiel 1 (Synthese von Hybrid-TS1 nach der Zwei-Schritt Methode)

Bezogen auf Siliziumdioxid beträgt der Gehalt an nicht-hydrolysierbaren organischen Liganden 0,5 Mol-%.

Als Ausgangsmaterialien wurde folgende Substanzen verwendet:

| | |
|---|---|
| Si-Quellen: | Tetraethylorthosilicat (TEOS, erhältlich von der Firma Merck) Methyltrimethoxysilan (MTMS, erhältlich von der Firma Merck) |
| | |
| Ti-Quelle: | Tetrabutylorthotitanat (TBOT, erhältlich von der Firma *Aldrich)* |
| Templat: | Tetrapropylammoniumhydroxid (TPAOH, erhältlich von der Firma SACHEN, HH) |
| Wasser: | Kationen freies Wasser (Kationen < 10 ppm) |
| | |
| Edukte | Einsatzmengen |
| TEOS | 137,78 g |
| MTMS | 0,46 g |
| HCl, 0,05 mol/l | 47,8 g |
| TBOT | 5,72 g |
| Isopropanol | 33,3 g |
| TPAOH (Base) | 10-12 ml |
| TPAOH (Templat) | 32 g |

### Hydrolyse der Siliziumkomponente

Zu einer gut gerührten Vorlage von TEOS und MTMS in einem 250 ml Rundkolben wurde die wässrige HCl-Lösung innerhalb von 5 min zudosiert und etwa 1 h lang gerührt. Dabei wurde eine maximale Temperatur von 69°C nach 39 min erreicht. Nach der vollständigen Hydrolyse des TEOS wurde die Mischung im Eisbad auf 1-2°C abgekühlt; dieser Vorgang dauerte etwa 40 min.

### Einbau der Ti-Spezies in das Netzwerk

Eine Lösung von 5.72 g TBOT, gelöst in 33,3 g Isopropanol wurde nun mittels einer Syringe-Pumpe sehr langsam zur Mischung zudosiert. Bei 11 ml/h dauerte die Dosierung 4,5 h. Dabei musste darauf geachtet werden, dass die Temperatur der Mischung nicht über 3°C steigt und mit maximaler Intensität gerührt wird, da sonst das Ausfallen von Anatas begünstigt wird. Nach beendeter Zugabe der Titankomponente musste die klare Lösung noch 1 h lang unter den gleichen Bedingungen weitergerührt werden, um eine vollständige Kondensation der Titanspecies im Hybrid-Netzwerk zu gewährleisten. Danach waren die freien Ti-OH Gruppen vollständig mit Silikaten abgesättigt, so dass die Reaktion ohne die Gefahr eines Anatasniederschlages beschleunigt werden konnte. Die klare Lösung wurde nun innerhalb von 30 min auf RT erwärmt.

### Basische Gelierung

Ca. 10 ml 20 %ige TPAOH-Lösung wurden mittels einer Syringe Pumpe mit einer Dosiergeschwindigkeit von 10 ml/h zu der gut gerührten Mischung dosiert. Nach 32 min wurde der Gelpunkt erreicht. Innerhalb weiterer 10 min veränderte sich der klare Gelkörper von flexibel zu spröde. Nach weiteren 60 min wurde der Gelkörper im Mixer zerkleinert und anschließend bei 12 h bei 110°C im Trockenschrank bei 300 mbar getrocknet. Das getrocknete Gel wurde nun auf 100-160 Mikrometer im Mörser zerkleinert.

### Zeolithbildung

20 g des getrockneten und zerkleinerten Pulver wurden in einem mit Teflon ausgekleideten Autoklaven vorgelegt, mit 32 g 20 %iger TPAOH-Lösung (1,6fache Gewichtsmenge) intensiv vermischt (Pulver muss gleichmäßig mit Flüssigkeit benetzt sein) unter Eigendruck bei 170°C 12 h in den Trockenschrank gestellt (Hydrothermal Synthese). Nach Abkühlung auf RT wurde der Reaktorinhalt mit VE-Wasser aus dem Reaktor gespült und in der Zentrifuge (5 min, 3000 Umin-1) die Feststoffphase von Flüssigphase getrennt. Der Feststoff wurde noch dreimal mit 30 ml alkalifreiem VE-Wasser gewaschen. Nach der Wäsche wurde das Produkt 4 h bei 110°C getrocknet und anschließend in zwei Varianten getempert und kalziniert:

**Hybrid-TS1 550:** innerhalb einer Stunde wurde das getrocknete amorphe Hybrid-Gel im Muffelofen unter N₂-Strom (250 1/h) auf 550°C erwärmt. Man hielt eine weitere Stunde unter N₂-Strom bei 550°C und kalzinierte anschließend 15 h bei 550°C unter Luftzufuhr (100 l/h).

**Hybrid-TS1 400short:** innerhalb einer Stunde wurde das getrocknete amorphe Hybrid-Gel im Muffelofen unter N₂-Strom (250 1/h) auf 400°C erwärmt. Man hielt eine weitere Stunde unter N₂-Strom bei 400°C und kalzinierte anschließend 15 h bei 400°C unter Luftzufuhr (100 l/h).

**Hybrid-TS1 400long:** innerhalb einer Stunde wurde das getrocknete amorphe Hybrid-Gel im Muffelofen unter N₂-Strom (250 l/h) auf 400°C erwärmt. Man hielt eine weitere Stunde unter N₂-Strom bei 400°C und kalzinierte anschließend 30h bei 400°C unter Luftzufuhr (100 l/h).

### Beispiel 1

Zur Reaktion wurden 0,2 g Hybrid-TS1 550 pulverförmiger Katalysator - suspendiert in einem Methanol/Wasser Gemisch (15 g Methanol, 5 g 30 %ige Wasserstoffperoxidlösung in Wasser) - bei 50°C und 3 bar vorgelegt. Die Suspension wurde mittels eines Magnetrührkerns mit 800 U/min⁻¹ gerührt. Die Eduktgase wurden durch eine 1 mm 0,2 mm Kapillare direkt in die Suspension eindosiert (Tauchung). Zur Durchführung der Oxidationsreaktionen mit 0,2 g Katalysator wurden 0,252 l/h C₃H₆ und 3,96 l/h N₂ (entspricht 6 % Propen in N₂) in die Flüssigphase eingeperlt.

In einem Test gemäß der Testvorschrift wurde eine konstante PO-Selektivitäten von 95 % erreicht. Die PO-Konzentration in der austretenden Gasphase lag bei 5 %. Die PO-Produktionsrate blieb so lange gleich, bis die H₂O₂-Konzentration in der Flüssigphase auf unter 4 % abgesagt war.

### Beispiel 2

Zur Reaktion wurden 0,2 g Hybrid-TS1 400short pulverförmiger Katalysator - suspendiert in einem Methanol/Wasser Gemisch (15 g Methanol, 5 g 30 %ige Wasserstoffperoxidlösung in Wasser) - bei 50°C und 3 bar vorgelegt. Die Versuchsdurchführung erfolgte analog Beispiel 1.

In einem Test gemäß der Testvorschrift wurde eine konstante PO-Selektivitäten von 95 % erreicht. Die PO-Konzentration in der austretenden Gasphase lag bei 6,1 %. Die PO-Produktionsrate blieb so lange gleich, bis die H₂O₂-Konzentrati in der Flüssigphase auf unter 4 % abgesackt war.

### Beispiel 3

Zur Reaktion wurden 0,2 g *Hybrid-TS1 400long* pulverförmiger Katalysator - suspendiert in einem Methanol/Wasser Gemisch (15 g Methanol, 5 g 30 %ige Wasserstoffperoxidlösung in Wasser) - bei 50°C und 3 bar vorgelegt. Die Versuchsdurchführung erfolgte analog Beispiel 1.

In einem Test gemäß der Testvorschrift wurde eine konstante PO-Selektivitäten von 95 % erreicht. Die PO-Konzentration in der austretenden Gasphase lag bei 6,5 %. Die PO-Produktionsrate blieb so lange gleich, bis die H₂O₂-Konzentration in der Flüssigphase auf unter 4 % abgesackt war.

### Beispiel 4

Cyclohexen wurde anstelle von Propen als ungesättigter Kohlenwasserstoff ausgewählt. Zur partiellen Oxidation von Cyclohexen wurde ein Katalysator analog Beispiel 3 verwendet. Cyclohexen wurde mit Hilfe eines Verdampfers in die Flüssigphase kontinuierlich gebracht.

In einem Test gemäß der Testvorschrift wurde eine konstante Epoxid-Selektivitäten von 93 % erreicht. Die Epoxid-Konzentration in der austretenden Gasphase lag bei 4 %. Die Cyclohexenoxid-Produktionsrate blieb so lange gleich, bis die H₂O₂-Konzentration in der Flüssigphase auf unter 5 % abgesackt war.

## Patentansprüche

1. Ein Katalysator enthaltend
a) Titan in gebundener Form,
b) nichtkristallines Siliziumdioxid und
c) mindestens eine kristalline Silikatphase, die eine Zeolithstruktur aufweist,
wobei das nichtkristalline Siliziumdioxid auf mindestens einer der kristallinen Silikatphasen, die eine Zeolithstruktur aufweisen, aufgebracht ist,
und wobei Titan in mindestens eine Zeolithstruktur tetraedisch eingebaut ist.
und wobei mindestens eine der kristallinen Silikatphasen, die eine Zeolithstruktur aufweisen, Silizium-Kohlenstoff-Bindungen enthält, mit denen nicht hydrolytisch abtrennbare organische Gruppen R an Silizium gebunden sind.

2. Ein Katalysator nach Anspruch 1 enthaltend zusätzlich
d) weiteres Silizium in elementarer oder in gebundener Form, das nicht in Form des nichtkristallinen Siliziumdioxides und auch nicht in Form einer kristallinen Silikatphase, die eine Zeolithstruktur aufweist, vorliegt,

3. Der Katalysator nach Anspruch 1 oder 2, wobei mindestens eine der kristallinen Silikatphasen, die eine Zeolithstruktur aufweisen, eine Zeolithstruktur aufweist, die ausgewählt ist aus der Gruppe bestehend aus MFI, MEL, BEA, MOR und einer Mischstruktur aus zweien oder mehreren dieser genannten Zeolithstrukturen.

4. Der Katalysator nach einem der Ansprüche 1 bis 3, wobei die kristalline Silikatphase, die Silizium-Kohlenstoff-Bindungen enthält, ein organisch modifiziertes Glas ist und im kristallinen Netzwerk terminale und/oder verbrückende organische Gruppen enthalten.

5. Der Katalysator nach einem der Ansprüche 1 bis 4, wobei die organischen Gruppen R in einer Menge von 0,01 bis 5 Gew.-% vorhanden sind, bezogen auf die Menge der kristallinen Silikatphasen, die eine Zeolithstruktur aufweisen und die Silizium-Kohlenstoff-Bindungen enthalten, mit denen nicht hydrolytisch abtrennbare organische Gruppen R an Silizium gebunden sind.

6. Ein Verfahren zur Herstellung des Katalysators nach einem der Ansprüche 1 bis 5, umfassend
i) das Zusammenbringen der Komponenten a) bis c), so dass eine Zusammensetzung vorliegt, die a) bis c) enthält, und
ii) das Kalzinieren der Zusammensetzung bei einer Temperatur von 100 bis 550°C.

7. Das Verfahren nach Anspruch 6 umfassend zusätzlich
iii) das Behandeln der Zusammensetzung mit einer sauren Wasserstoffperoxidlösung nach dem Kalzinieren.

8. Das Verfahren nach Anspruch 7 umfassend zusätzlich
iv) das Behandeln der Zusammensetzung mit einer Alkalilösung nach dem Behandeln mit einer sauren Wasserstoffperoxidlösung.

9. Der Katalysator erhältlich nach dem Verfahren nach einem der Ansprüche 6 bis 8.

10. Ein Verfahren zur Herstellung eines Epoxides aus einer Verbindung, die eine Kohlenstoff-Kohlenstoff-Doppelbindung enthält (bevorzugt aus Propen), umfassend die Umsetzung der Verbindung, die eine Kohlenstoff-Kohlenstoff-Doppelbindung enthält, mit Wasserstoffperoxid in Gegenwart des Katalysators nach einem der Ansprüche 1 bis 5 oder 9.
